# EUROPEAN PATENT SPECIFICATION

(11) **EP 3 070 450 B1**
(45) Date of publication and mention of the grant of the patent: **09.01.2019**
(21) Application number: 14861214.6
(22) Date of filing: 15.05.2014
(51) Int. Cl.: G01L 19/10, G01L 13/02, A61M 16/00

(54) **PRESSURE INDICATOR**
DRUCKANZEIGER
INDICATEUR DE PRESSION

(30) Priority: 14.11.2013 CN 201320727634 U; 14.11.2013 CN 201310575100
(43) Date of publication of application: 21.09.2016
(73) Proprietor: Zou, Dewei, Tianjin 300385 (CN)
(72) Inventor: Zou, Dewei, Tianjin 300385 (CN)
(74) Representative: Lermer, Christoph
(86) International application number: PCT/CN2014/077546
(87) International publication number: WO 2015/070579

(56) References cited:
- CN-A- 102 049 079
- CN-A- 103 301 548
- CN-A- 103 592 075
- CN-U- 202 138 378
- CN-U- 202 853 843
- CN-U- 203 587 275
- CN-Y- 2 478 090
- CN-Y- 2 755 590
- CN-Y- 2 759 496
- CN-Y- 201 179 260
- US-A1- 2006 107 745
- US-A1- 2008 004 541
- US-A1- 2008 228 087
- US-A1- 2009 145 236

## Description

### FIELD OF THE INVENTION

This invention relates to apparatus used to measure and indicate pressure, which is mainly applied to the internal pressure measurement and indication of gasbag and water sac in medical apparatus and instrument, and more particularly to a pressure indicator used to measure and indicate the pressure in laryngeal mask, tracheal intubation and catheter.

### BACKGROUND OF THE INVENTION

Gasbag and water sac are widely applied in field of medical apparatus and instruments, such as tracheal tube, laryngeal mask, catheter, and the like medical apparatus and instruments. Gasbag and water sac are used to seal body cavity and fix device. For the sake of the safety of the apparatus, it is very important to measure the pressure in gasbag and water sac, and the pressure is required to be measured at any moment clinically.

Pressure indicators and the clinical application value thereof attract great attention of medical workers and medical apparatus and instruments industry. As a normal pressure indicator has a simple structure and function, therefore, recently, to meet the requirements of the continuous development of healthcare, the structure of pressure indicator is increasingly improved. Patent literatures relating to the structure of pressure indicators are reported as follows:
1. China Utility Model Patent Specification, patent number: ZL200820074424.2 (issue date: January 14th, 2009), disclosed a pressure indicator for gasbag. The structure includes a transparent and hollow cavity which has a graduation line, an elastic sac with one end closed and another end open, a check valve and connecting joints. The top of the hollow cavity connects with an air inflation/exhaust port via the check valve which is sealed to the top. The elastic sac is arranged inside the hollow cavity. The opening end of the sac is sealed and fixed on connecting joints, and the inner cavity of the sac communicates with the air. The interior and exterior of the elastic sac forms a pressure zone inside the sac and a pressure zone outside the sac, respectively. The elastic sac, coordinating with the graduation line of hollow cavity, produces a transformation displacement as the inner and outer pressures of the sac change, and a pressure indicator color code is disposed outside the measuring gasbag.
   The advantages of the pressure indicator for gasbag are: highly sensitive, small in size, light in weight, visual, enabling to be directly disposed on an air channel equipment, easy to use, low-cost. The pressure indicator for gasbag is especially applicable for medical apparatus and instruments with gasbag such as laryngeal mask, tracheal intubation with gasbag and the like.
2. International Application PCT/US2001/012027 2001.4.13, China Invention Patent Specification (Chinese Publication No.: CN100333808C, issue date: August 29th, 2007) disclosed a visible inflated pressure indicator and surgical intubation of the same. The invention provides an indicator which can visually indicate the pressure of fluid in a pressure source. The indicator includes a visible inflation body with a notch, which has a relative boundary part, and when the inflation body is in an uninflated state, the relative boundary part tends to divide from a gap. The inflation body also includes a hole which connects the inflation body with the pressure source. Therefore, when the inflation body is inflated, the relative boundary part has relative movement along a certain direction so as to reduce the size of the gap, and indicate that the expected pressure in the pressure source has reached. The indicator is particularly used to indicate that the expected pressure in an inflation envelop of the intubation inside the tube has reached.
   The advantage of the pressure indicator: providing a simple and visible pressure indication in the pressure source. It is particularly useful to ensure whether the inflation envelope is overinflated under the condition that expensive pressure meters or devices are inapplicable, for the pressure indicator being accurate, simple to use and produce.
3. China Utility Model Patent Specification, patent number: ZL03232298.4 (issue date: August 4th, 2004) disclosed a pressure testing indicator, including: a cylinder block, a seal ring and valve actuation components. A top cover which is transparent in the middle is arranged on the cylinder block. Inside the cylinder block, the valve actuation components with air-in via holes are connected via screw thread. Elastic diaphragms are arranged between the valve actuation components and the top cover. A gas pressure indicator drum is disposed on the elastic diaphragm, and a spring is arranged between the gas pressure indicator drum and the top cover. The surface of the elastic diaphragm and gas pressure indicator drum are both provided with patterns and colors to indicate the variation of pressure. Indicatrix is positioned on the lower edge of an opaque part of the top cover, and the graduation line is arranged on the surface of the gas pressure indicator drum.
   The advantages of the pressure testing indicator are: simple structure, low production cost, ease of use, and visual indication of the pressure variation in pressure and pneumatic equipment. The pressure testing indicator is particularly applicable for accurate pressure measurement of gas pressure in auto tire.
   However, the above three pressure indicators all have the following defects: (1) small scope of pressure indication and poor adaptability; (2) simple structure, yet poor accuracy, failing to meet the requirements of accurate pressure measurement. Document CN 2 755 590 Y discloses a pressure indicator comprising an elastic pressure-sensing diaphragm. Document US 2008/0190189 A1 discloses also a pressure indicator that is coaxial or concentric with a fill valve and comprises an elastic pressure-sensing diaphragm; such pressure indicator is designed primarily for automobile tires, but could be used for other inflatable articles.

### SUMMARY OF THE INVENTION

In view of the above-described problems, it is one objective of the invention to provide a pressure indicator which features a compact structure, accurate indication and wide application.

To achieve the above objective, in accordance with one embodiment of the invention, there is provided a pressure indicator, comprising: a check valve; an upper cover, the upper cover comprising an inner wall, a circular groove, and a positioning bulge; an intermediate member, the intermediate member comprising a positioning groove, a lower port; an elastic pressure-sensing diaphragm, the elastic pressure-sensing diaphragm comprising a circumferential flange, a lower surface; a transmission indication rod, the transmission indication rod comprising a support end, a connection rod, and an indication end; and a lower cover. The check valve is disposed in the upper cover, and the inner wall of the upper cover and the check valve are assembled to form an upper opening. The circular groove is disposed on a shoulder of the upper cover; an opening of an air channel which communicates with the air is disposed inside the circular groove. The positioning bulge of the upper cover is connected to the positioning groove of the intermediate member. The lower port of the intermediate member is bonded with the circumferential flange of the elastic pressure-sensing diaphragm. The lower surface of the elastic pressure-sensing diaphragm is provided with a groove configured to coordinate with the support end of the transmission indication rod. The connection rod is connected to a top and bottom of the support end, and the indication end comprises holes on two sides thereof.

The air channel is formed by and runs through the upper cover and the intermediate member.

At least one surface of the elastic pressure-sensing diaphragm is provided with at least one circular or oval circumferential reinforcing bar and at least one longitudinal reinforcing bar which diffuses from center to all round. The longitudinal reinforcing bar is between 2 and 16 in number. The longitudinal reinforcing bar and the circumferential reinforcing bar are uniformly distributed in a vertically crossing way; and a cross section of the circumferential reinforcing bar and the longitudinal reinforcing bar is designed to be waved, rectangular, triangular or other geometrical shapes.

The elastic pressure-sensing diaphragm is a circular chip, oval chip or chip with other geometrical shapes, with a thickness thereof of between 0.1 mm and 2.0 mm.

The elastic pressure-sensing diaphragm is made of rubber, silastic, thermoplastic elastomer (TPE) or other high elastic macromolecule material.

Compared with existing technologies, advantages of the pressure indicator according to embodiments of the invention are given below:
1. Wide range of pressure indication, specifically, the water column ranges from 10 cm to 100 cm.
2. High indication accuracy and high differentiability.
3. High adaptability, enabling it to be used in the monitoring and measurement of air pressure and hydraulic pressure.

### BRIEF DESCRIPTION OF THE DRAWINGS

FIG. 1 is a cross-sectional view of a pressure indicator in accordance with one embodiment of the invention;
FIG. 2 is a sectional view of an upper cover of a pressure indicator in accordance with one embodiment of the invention;
FIG. 3 is a top view of an upper cover of a pressure indicator in accordance with one embodiment of the invention;
FIG. 4 is a bottom view of an upper cover of a pressure indicator in accordance with one embodiment of the invention;
FIG. 5 is a schematic diagram of an intermediate member of a pressure indicator in accordance with one embodiment of the invention;
FIG. 6 is a schematic diagram of an elastic pressure-sensing diaphragm of a pressure indicator in accordance with one embodiment of the invention; and
FIG. 7 is a cross-sectional view of an elastic pressure-sensing diaphragm of a pressure indicator in accordance with one embodiment of the invention.

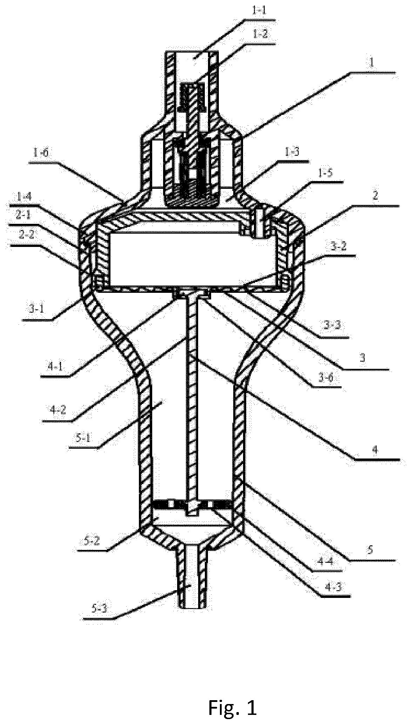

Legends, FIG. 1: 1. Upper cover; 1-1. Upper opening; 1-2. Check valve; 1-3. Lower space; 1-4. Gap; 1-5. Air channel; 1-6. Circular groove; 1-7. Positioning bulge; 2.Intermediate member; 2-1. Bonding gap; 2-2. Lower port; 3. Elastic pressure-sensing diaphragm; 3-1. Circumferential flange; 3-2. Upper surface; 3-3. Lower surface; 3-6. Groove; 4. Transmission indication rod; 4-1. Support end; 4-2. Connection rod; 4-3. Hole; 4-4. Indication end; 5. Lower cover; 5-1. Upper space; 5-2. Lower space; 5-3. Lower mouth.

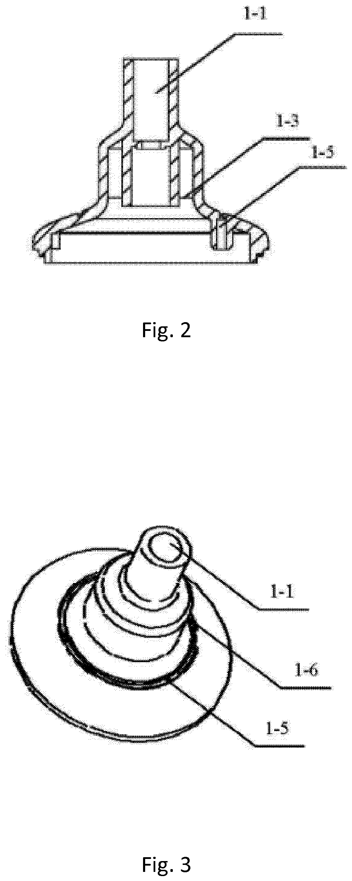

FIG. 2: 1-1. Upper opening; 1-3. Lower space; 1-5. Air channel.

FIG. 3: 1-1. Upper opening; 1-5. Air channel; 1-6. Circular groove.

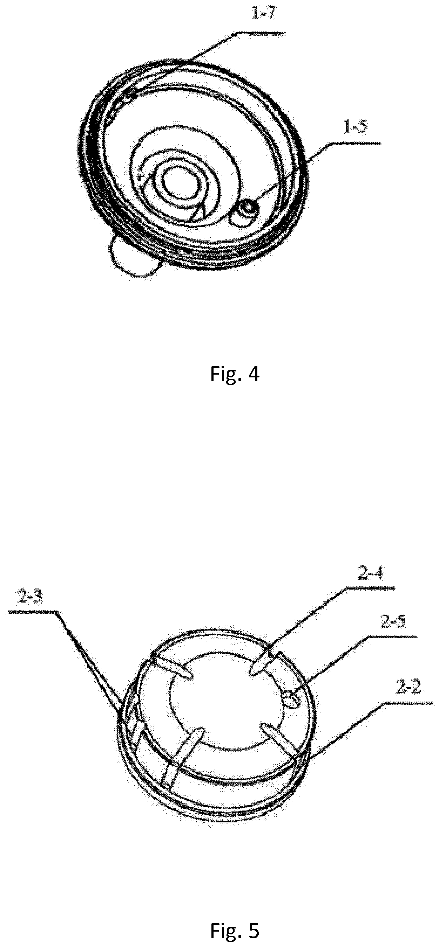

FIG. 4: 1-5. Air channel; 1-7. Positioning bulge.

FIG. 5: 2-2. Lower port; 2-3. Positioning groove; 2-4. Airway; 2-5. Air channel.

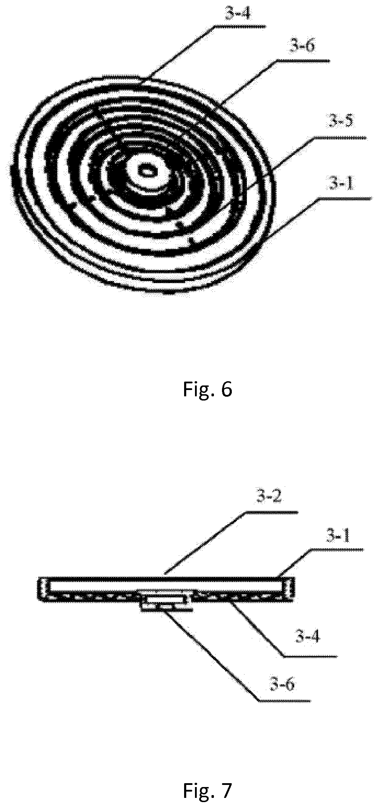

FIG. 6: 3-1. Binding groove; 3-4. Circumferential reinforcing bar; 3-5. Longitudinal reinforcing bar; 3-6. Groove.

FIG. 7: 3-1. Binding groove; 3-2. Upper surface; 3-4. Circumferential reinforcing bar; 3-6. Groove.

### DETAILED DESCRIPTION OF THE EMBODIMENTS

For further illustrating the invention, experiments detailing a pressure indicator are described below. It should be noted that the following examples are intended to describe and not to limit the invention.

### Example

As shown in FIG. 1, a pressure indicator comprises: an upper cover 1, an intermediate member 2, an elastic pressure-sensing 3, a transmission indication rod 4, and a lower cover 5. As shown in FIGs. 2-4, a check valve 1-2 is disposed in the upper cover 1, and the inner wall of the upper cover and the check valve 1-2 are assembled to form the upper opening 1-1. A circular groove 1-6 is disposed on the shoulder of the upper cover. The opening of the air channel 1-5 which communicates with the air is disposed inside the circular groove 1-6. The positioning bulge 1-7 of the upper cover 1 is connected to the positioning groove 2-3 of the intermediate member 2.

The lower port 2-2 of the intermediate member 2 is bonded with the circumferential flange 3-1 of the elastic pressure-sensing diaphragm 3. A groove 3-6 which coordinates with the support end 4-1 of the transmission indication rod 4 is disposed on the lower surface 3-3 of the elastic pressure-sensing diaphragm 3, as shown in FIG. 1.

The transmission indication rod 4 comprises: support end 4-1, connection rods 4-2 respectively connected to the top and bottom of the support end 4-1, indication end 4-4 and holes 4-3 on two sides, as shown in FIG. 1.

As shown in FIG. 1, the air channel 1-5 is formed by and runs through the upper cover 1 and the intermediate member 2.

As shown in FIGs. 6-7, at least one surface of the elastic pressure-sensing diaphragm 3 is provided with at least one circular or oval circumferential reinforcing bar 3-4 and at least one longitudinal reinforcing bar 3-5 which diffuses from center to all round. The longitudinal reinforcing bar 3-5 is between 2 and 16 in number, which is 4 in this example. The longitudinal reinforcing bar 3-5 and the circumferential reinforcing bar 3-4 are equally distributed in a vertically crossing way. The cross section of the circumferential reinforcing bar 3-4 and the longitudinal reinforcing bar 3-5 is designed to be waved, rectangular, triangular or other geometrical shapes. In this example, the cross section of the circumferential reinforcing bar 3-4 and the longitudinal reinforcing bar 3-5 is waved.

The elastic pressure-sensing diaphragm 3 is made of rubber, silastic, thermoplastic elastomer (TPE) or other high elastic macromolecule material. In the example, rubber is employed.

An application method of the pressure indicator is summarized as follows.

Pressure measurement of a gas bag is taken as an example. As shown in FIG. 1, while the pressure indicator is in use, the lower mouth 5-3 is in airtight connection with the gas bag via an inflating tube. A syringe needle is applied to inflate the pressure indicator via the upper opening 1-1, and the gas enters the lower space 1-3 via the check valve 1-2, as shown in FIG. 2. Then, the gas passes through the gap 1-4 in FIG. 1 and enters the upper space 5-1 of the lower cover 5. On the one hand, as shown in FIG. 1, the gas passes through the holes 4-3 beneath the transmission indication rod 4, enters the lower space 5-2, and inflates the gas bag via the lower mouth 5-3. On the other hand, as shown in FIG. 1, the gas pushes the lower surface 3-3 of the elastic pressure-sensing diaphragm 3 to move upward, and pushes upward the transmission indication rod 4 in the same time, so that the change of the indication end 4-4 indicates the pressure change and that the number is accessible on the calibration line outside the lower cover. The upper surface of the elastic pressure-sensing diaphragm 3 communicates with the air via the air channel 1-5. The elastic pressure-sensing diaphragm 3 always indicates the pressure difference between the gas bag and atmosphere, and the structure of the pressure indicator guarantees the accuracy of the measurement.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the true scope of the invention.

## Claims

1. A pressure indicator, comprising:
a) a check valve (1-2);
b) an upper cover (1), the upper cover comprising an inner wall,
c) an elastic pressure-sensing diaphragm (3), the elastic pressure-sensing diaphragm comprising a circumferential flange (3-1) and a lower surface (3-3);
d) a transmission indication rod (4), the transmission indication rod comprising a support end (4-1), a connection rod(4-2), and an indication end (4-4), wherein the connection rod (4-2) is connected to a top and bottom of the support end (4-1);
e) a lower cover (5); and
f) an opening of an air channel (1-5) which communicates with the atmospheric air,
**characterized in that**
the upper cover (1) comprises a circular groove (1-6), and a positioning bulge (1-7); and the pressure indicator further comprises an intermediate member (2), the intermediate member comprising a positioning groove (2-3) and a lower port (2-2); wherein the check valve (1-2) is disposed in the upper cover (1), and the inner wall of the upper cover and the check valve (1-2) are assembled to form an upper opening (1-1); the circular groove (1-6) is disposed on a shoulder of the upper cover (1); the opening of the air channel (1-5) which communicates with the atmospheric air is disposed inside the circular groove (1-6); the positioning bulge (1-7) of the upper cover (1) is connected to the positioning groove (2-3) of the intermediate member (2);
the lower port (2-2) of the intermediate member (2) is bonded with the circumferential flange (3-1) of the elastic pressure-sensing diaphragm (3); the lower surface (3-3) of the elastic pressure-sensing diaphragm (3) is provided with a groove (3-6) configured to coordinate with the support end (4-1) of the transmission indication rod (4);
and the indication end (4-4) comprises holes (4-3) on two sides thereof.

2. The pressure indicator of claim 1, **characterized in that** the air channel (1-5) is formed by and runs through the upper cover (1) and the intermediate member (2).

3. The pressure indicator of claim 1, **characterized in that** at least one surface of the elastic pressure-sensing diaphragm (3) is provided with at least one circular or oval circumferential reinforcing bar (3-4) and at least one longitudinal reinforcing bar (3-5) which diffuses from the center to all round; the number of longitudinal reinforcing bars (3-5) is between 2 and 16; the longitudinal reinforcing bar (3-5) and the circumferential reinforcing bar (3-4) are uniformly distributed in a vertically crossing way; and a cross section of the circumferential reinforcing bar (3-4) and the longitudinal reinforcing bar (3-5) is designed to be waved, rectangular, triangular or other geometrical shapes.

4. The pressure indicator of claim 1, **characterized in that** the elastic pressure-sensing diaphragm (3) is a circular chip, oval chip or chip with other geometrical shapes, with a thickness thereof of between 0.1 mm and 2.0 mm.

5. The pressure indicator of claim 1, **characterized in that** the elastic pressure-sensing diaphragm (3) is made of rubber, silastic, thermoplastic elastomer (TPE) or other high elastic macromolecule material.

## Patentansprüche

1. Druckanzeiger, umfassend:
a) ein Rückschlagventil (1-2);
b) eine obere Abdeckung (1), wobei die obere Abdeckung eine Innenwand aufweist,
c) eine elastische Druckmessmembran (3), wobei die elastische Druckmessmembran einen umlaufenden Flansch (3-1), und eine untere Oberfläche (3-3) umfasst;
d) eine Übertragungs-Anzeigestange (4), wobei die Übertragungs-Anzeigestange ein Stützende (4-1), eine Verbindungsstange (4-2), und ein Anzeigeende (4-4) umfasst, wobei die Verbindungsstange (4-2) mit einer Oberseite und einer Unterseite des Stützendes (4-1) verbunden ist;
e) eine untere Abdeckung (5); und
f) eine Öffnung eines Luftkanals (1-5), der mit der Atmosphärenluft in Verbindung steht, **dadurch gekennzeichnet**
die obere Abdeckung (1) eine kreisförmige Nut (1-6) und eine Positionierungswulst (1-7) umfasst; und
der Druckindikator ferner ein Zwischenelement (2) umfasst, wobei das Zwischenelement eine Positionierungsnut (2-3) und einen unteren Anschluss (2-2) umfasst; wobei das Rückschlagventil (1-2) in der oberen Abdeckung (1) angeordnet ist und die Innenwand der oberen Abdeckung und das Rückschlagventil (1-2) zusammengebaut sind, um eine obere Öffnung (1-1) zu bilden; die kreisförmige Nut (1-6) ist an einer Schulter der oberen Abdeckung (1) angeordnet; die Öffnung des Luftkanals (1-5), die mit der Atmosphärenluft in Verbindung steht, ist innerhalb der kreisförmigen Nut (1-6) angeordnet; der Positionierungswulst (1-7) der oberen Abdeckung (1) ist mit der Positionierungsnut (2-3) des Zwischenelements (2) verbunden;
der untere Anschluss (2-2) des Zwischenelements (2) ist mit dem Umfangsflansch (3-1) der elastischen Druckmessmembran (3) verbunden; die untere Oberfläche (3-3) der elastischen Druckmessmembran (3) ist mit einer Nut (3-6) versehen, die konfiguriert ist, um mit dem Stützende (4-1) der Übertragungs-Anzeigestange (4) aufeinander abgestimmt zu sein;
und das Anzeigeende (4-4) weist an zwei Seiten Löcher (4-3) auf.

2. Druckanzeiger nach Anspruch 1, **dadurch gekennzeichnet, dass** der Luftkanal (1-5) durch die obere Abdeckung (1) und das Zwischenelement (2) gebildet ist und durch diese verläuft.

3. Druckanzeiger nach Anspruch 1, **dadurch gekennzeichnet, dass** mindestens eine Oberfläche der elastischen Druckmessmembran (3) mit mindestens einem kreisförmigen oder ovalen umlaufenden Bewehrungsstab (3-4) und mindestens einem Längsbewehrungsstab versehen ist (3-5), der sich von der Mitte zu allen Rundungen ausbreitet; die Anzahl der Längsbewehrungsstäbe (3-5) zwischen 2 und 16 liegt; der Längsbewehrungsstab (3-5) und der Umfangsbewehrungsstab (3-4) in einer sich vertikal kreuzenden Weise gleichförmig verteilt sind; und ein Querschnitt des Umfangsbewehrungsstabs (3-4) und des Längsverstärkungsstabs (3-5) ausgebildet ist, um wellenförmig, rechteckig, dreieckig oder in anderen geometrischen Formen zu sein.

4. Druckanzeiger nach Anspruch 1, **dadurch gekennzeichnet, dass** die elastische Druckmessmembran (3) ein kreisförmiger Chip, ovaler Chip oder Chip mit anderen geometrischen Formen ist, mit einer Dicke von zwischen 0,1 mm und 2,0 mm.

5. Druckanzeiger nach Anspruch 1, **dadurch gekennzeichnet, dass** die elastische Druckmessmembran (3) aus Gummi, Silastic, thermoplastischem Elastomer (TPE) oder einem anderen hochelastischen Makromolekülmaterial besteht.

## Revendications

1. Indicateur de pression, comprenant :
a) un clapet de non-retour (1-2) ;
b) un couvercle supérieur (1), le couvercle supérieur comprenant une paroi intérieure,
c) une membrane de détection de pression élastique (3), la membrane de détection de pression élastique comprenant une collerette circonférentielle (3-1) et une surface inférieure (3-3) ;
d) une tige d'indication de transmission (4), la tige d'indication de transmission comprenant une extrémité de support (4-1), une tige de liaison (4-2) et une extrémité d'indication (4-4), dans lequel la tige de liaison (4-2) est reliée à un haut et un bas de l'extrémité de support (4-1) ;
e) un couvercle inférieur (5) ; et
f) une ouverture d'un canal d'air (1-5) qui communique avec l'air atmosphérique,
**caractérisé en ce que**
le couvercle supérieur (1) comprend une rainure circulaire (1-6) et un bombement de positionnement (1-7) ; et
l'indicateur de pression comprend en outre un élément intermédiaire (2), l'élément intermédiaire comprenant une rainure de positionnement (2-3) et un orifice inférieur (2-2) ;
dans lequel le clapet de non-retour (1-2) est disposé dans le couvercle supérieur (1), et la paroi intérieure du couvercle supérieur et le clapet de non-retour (1-2) sont assemblés pour former une ouverture supérieure (1-1) ; la rainure circulaire (1-6) est disposée sur un épaulement du couvercle supérieur (1) ;
l'ouverture du canal d'air (1-5) qui communique avec l'air atmosphérique est disposée à l'intérieur de la rainure circulaire (1-6) ; le bombement de positionnement (1-7) du couvercle supérieur (1) est relié à la rainure de positionnement (2-3) de l'élément intermédiaire (2) ;
l'orifice inférieur (2-2) de l'élément intermédiaire (2) est lié à la collerette circonférentielle (3-1) de la membrane de détection de pression élastique (3) ; la surface inférieure (3-3) de la membrane de détection de pression élastique (3) est dotée d'une rainure (3-6) configurée pour la coordination avec l'extrémité de support (4-1) de la tige d'indication de transmission (4) ;
et l'extrémité d'indication (4-4) comprend des trous (4-3) sur les deux côtés de celle-ci.

2. Indicateur de pression selon la revendication 1, **caractérisé en ce que** le canal d'air (1-5) est formé par, et passe à travers le couvercle supérieur (1) et l'élément intermédiaire (2).

3. Indicateur de pression selon la revendication 1, **caractérisé en ce qu'**au moins une surface de la membrane de détection de pression élastique (3) est dotée d'au moins une barre de renforcement circonférentielle circulaire ou ovale (3-4) et d'au moins une barre de renforcement longitudinale (3-5) qui s'étend à partir du centre partout autour ;
le nombre de barres de renforcement longitudinales (3-5) est compris entre 2 et 16 ;
la barre de renforcement longitudinale (3-5) et la barre de renforcement circonférentielle (3-4) étant réparties uniformément de manière traversante verticalement ; et une section transversale de la barre de renforcement circonférentielle (3-4) et la barre de renforcement longitudinale (3-5) est conçue pour être ondulée, rectangulaire, triangulaire, ou avec d'autres formes géométriques.

4. Indicateur de pression selon la revendication 1, **caractérisé en ce que** la membrane de détection de pression élastique (3) est une pastille circulaire, une pastille ovale ou une pastille avec d'autres formes géométriques, avec une épaisseur de celle-ci comprise entre 0,1 mm et 2,0 mm.

5. Indicateur de pression selon la revendication 1, **caractérisé en ce que** la membrane de détection de pression élastique (3) est constituée par du caoutchouc, silastic, un élastomère thermoplastique (TPE), ou un autre matériau macromoléculaire hautement élastique.
